# EUROPÄISCHE PATENTSCHRIFT

(11) **EP 0 731 080 B1**
(45) Veröffentlichungstag und Bekanntmachung des Hinweises auf die Patenterteilung: **27.05.1998**
(21) Anmeldenummer: 96103128.3
(22) Anmeldetag: 01.03.1996
(51) Int. Cl.: C07C 51/25, C07C 51/265, C07C 51/235, C07C 45/32

(54) **Weiterverwendung des Verdünnungsgases aus der Gasphasen-Partialoxidation einer organischen Verbindung**
Reutilisation of dilution gas from a partial oxidation in gaseous phase of an organic compound
Réutilisation du gaz de dilution à partir d'une oxidation partielle en phase gazeuse d'un composé organique

(30) Priorität: 10.03.1995 DE 19508531; 13.07.1995 DE 19525505
(43) Veröffentlichungstag der Anmeldung: 11.09.1996
(73) Patentinhaber: BASF AKTIENGESELLSCHAFT, 67056 Ludwigshafen (DE)
(72) Erfinder: Hefner, Werner, Dr., 68623 Lampertheim (DE); Machhammer, Otto, Dr., 67281 Kirchheim (DE); Neumann, Hans-Peter, Dr., 67067 Ludwigshafen (DE); Tenten, Andreas, Dr., 67487 Maikammer (DE); Ruppel, Wilhelm, Dr., 67227 Frankenthal (DE); Vogel, Herbert, Dr., 67069 Ludwigshafen (DE)

(56) Entgegenhaltungen:
- EP-A- 0 127 092
- EP-A- 0 128 404
- WO-A-92/03213
- DE-A- 2 425 939
- DE-A- 3 114 984
- GB-A- 2 097 476
- JOURNAL A, Bd. 25, Nr. 3, Juli 1984, ANTWERPEN, Seiten 165-167, XP002005910 J.REUMERS: "ENERGY CONSERVATION AT AMOCO CHEMICALS"

## Beschreibung

Die vorliegende Erfindung betrifft ein neues Verfahren der kontinuierlich betriebenen heterogen katalysierten Gasphasen-Partialoxidation einer organischen Verbindung in einem Oxidationsreaktor, dessen Beschickungsgasgemisch neben der partiell zu oxidierenden organischen Verbindung und molekularem Sauerstoff als Oxidationsmittel wenigstens ein sich unter den Bedingungen der heterogen katalysierten Gasphasen-Partialoxidation im wesentlichen inert verhaltendes Verdünnungsgas umfaßt, wobei das sich im wesentlichen inert verhaltende Verdünnungsgas wenigstens zum Teil aus brennbaren Gasen besteht.

Unter einer vollständigen Oxidation einer organischen Verbindung wird hier verstanden, daß die organische Verbindung unter reaktiver Einwirkung von molekularem Sauerstoff so umgesetzt wird, daß der in der organischen Verbindung insgesamt enthaltene Kohlenstoff in Oxide des Kohlenstoffs und der in der organischen Verbindung insgesamt enthaltene Wasserstoff in Oxide des Wasserstoffs umgewandelt wird. Alle davon verschiedenen Umsetzungen einer organischen Verbindung unter reaktiver Einwirkung von molekularem Sauerstoff werden hier als Partialoxidationen einer organischen Verbindung zusammengefaßt.

Im besonderen sollen hier unter Partialoxidationen solche Umsetzungen organischer Verbindungen unter reaktiver Einwirkung von molekularem Sauerstoff verstanden werden, bei denen die partiell zu oxidierende organische Verbindung nach beendeter Umsetzung wenigstens ein Sauerstoffatom mehr chemisch gebunden enthält als vor Durchführung der Partialoxidation.

Als ein sich unter den Bedingungen der heterogen katalysierten Gasphasen-Partialoxidation im wesentlichen inert verhaltendes Verdünnungsgas werden solche Verdünnungsgase verstanden, deren Bestandteile unter den Bedingungen der heterogen katalysierten Gasphasen-Partialoxidation - jeder Bestandteil für sich betrachtet - zu mehr als 95 mol-%, vorzugsweise zu mehr als 98 mol-%, unverändert erhalten bleiben.

Unter brennbaren Gasen werden hier solche Verbindungen verstanden, deren bei einem Ausgangsdruck von 1 bar und einer Ausgangstemperatur von 50 bis 100°C befindliche Mischungen mit Luft eine obere und eine untere Explosionsgrenze (Entzündungsgrenze) aufweisen, wobei sich die Ermittlung der Explosionsgrenzen auf eine Bestimmung in der Standard-Apparatur gemäß W. Berthold et al in Chem.-Ing. Tech. 56 (1984) Nr. 2, S.126-127 bezieht.

Unter Explosionsgrenzen werden dabei nachfolgende Grenzwerte gemäß DIN 51649 verstanden:

In einem Gemisch aus Luft und einem brennbaren Gas ist die Geschwindigkeit, mit der sich unter vorgegebenen Ausgangsbedingungen eine durch eine örtliche Zündquelle (z.B. glühender Platindraht) eingeleitete Verbrennung (Entzündung, Explosion) ausbreitet, vom Gehalt an brennbarem Gas abhängig. Sie ist bei einem bestimmten Gehalt am größten. Sowohl bei Verringerung als auch bei Erhöhung des Gehaltes an brennbarem Gas wird die Verbrennungsgeschwindigkeit kleiner, bis sich schließlich die Verbrennungsreaktion bei einem unteren und einem oberen Grenzwert für den Gehalt an brennbarem Gas gerade nicht mehr von der Zündquelle her ausbreitet. Diese beiden Grenzwerte sind die untere Explosionsgrenze und die obere Explosionsgrenze; der dazwischen liegende Bereich des Gehalts an brennbarem Gas ist der Explosionsbereich (Entzündungsbereich). Gase, deren entsprechende Gemische mit Luft bei jedem Mischungsverhältnis zu keiner sich von einer Zündquelle ausbreitenden Verbrennungsreaktion befähigt sind, sind nicht brennbare inerte Verdünnungsgase.

Beispiele für brennbare Gase sind H₂, NH₃, Methan, Ethan, Propan, Butan (n- und/oder iso-), Pentan(n-,iso-und/oder neo-), Propin, CO, Acetylen, Ethylen, Diethylether, Cyanwasserstoff und Schwefelwasserstoff. Weitere Beispiele zeigt die Tabelle auf Seite 1285 von Römpp Chemie Lexikon, Cm-G, Thieme Verlag, Stuttgart, 8. Auflage (199O). Beispiele für nicht brennbare Gase sind CO₂, H₂O ,N₂ und alle Edelgase.

Inwieweit sich ein brennbares Gas bezüglich einer bestimmten heterogen katalysierten Gasphasen-Partialoxidation inert verhält und damit als brennbarer Bestandteil des zu verwendenden im wesentlichen inerten Verdünnungsgases in Betracht kommt, kann der Fachmann mittels weniger Vorversuche ermitteln.

Es ist allgemein bekannt, daß durch partielle Oxidation verschiedener organischer Verbindungen mit molekularem Sauerstoff zahlreiche Grundchemikalien erzeugt werden können. Beispielhaft genannt seien die Umsetzung von Propylen zu Acrolein und/oder Acrylsäure (vgl. z.B. DE-A-2 351 151), die Umsetzung von tert.Butanol, iso-Buten, iso-Butan, iso-Butyraldehyd oder dem Methylether des tert.-Butanols zu Methacrolein und/oder Methacrylsäure (vgl. z.B. DE-A-2 526 238, EP-B-92 O97, EP-B-58 927, DE-A-4 132 263, DE-A-4 132 684 und DE-A-4 O22 212), die Umsetzung von Acrolein zu Acrylsäure, die Umsetzung von Methacrolein zu Methacrylsäure (vgl. z.B. DE-A-2 526 238), die Umsetzung von o-Xylol oder Naphthalin zu Phthalsäureanhydrid (vgl.z.B. EP-A 522871) sowie die Umsetzung von Butadien zu Maleinsäureanhydrid (vgl. z.B. DE-A-2 106 796 und DE-A-1 624 921), die Umsetzung von Indanen zu z.B. Anthrachinon (vgl. z.B. DE-A-2 025 430), die Umsetzung von Ethylen zu Ethylenoxid (vgl. z.B. DE-AS-1 254 137, DE-A-2 159 346, EP-A-372 972, WO-89/0710, DE-A-4 311 608 und Beyer, Lehrbuch der organischen Chemie, 17. Auflage (1973), Hirzel Verlag Stuttgart, S. 261), die Umsetzung von Butadien zu Vinyloxiran ( vgl. z.B. US-A-5 312 931), die Umsetzung von Propylen und/oder Acrolein zu Acrylnitril (vgl. z.B. DE-A-2 351 151), die Umsetzung von iso-Buten und/oder Methacrolein zu Methacrylnitril, die oxidative Dehydrierung von Kohlenwasserstoffen (vgl. Z.B. DE-A-2351151), etc.

Aus den vorgenannten Schriften ist ferner allgemein bekannt, daß partielle Oxidationen organischer Verbindungen in Anwendung von molekularem Sauerstoff als Oxidationsmittel in besonders selektiver Weise in der Gasphase an im festen Aggregatzustand befindlichen Katalysatoren durchgeführt werden können. Besonders häufig handelt es sich bei den Katalysatoren dabei um Oxidmassen oder um Edelmetalle (z.B. Ag). Die katalytisch aktive Oxidmasse kann neben Sauerstoff lediglich ein anderes Element oder mehr als ein anderes Element (Multimetalloxidmassen) enthalten. Besonders häufig kommen als katalytisch aktive Oxidmassen solche zur Anwendung, die mehr als ein metallisches, insbesondere übergangsmetallisches, Element umfassen. In diesem Fall spricht man von Multimetalloxidmassen. Üblicherweise sind Multielementoxidmassen keine einfachen physikalischen Gemische von Oxiden der elementaren Konstituenten, sondern heterogene Gemische von komplexen Polyverbindungen dieser Elemente.

Meist werden diese heterogen katalysierten Gasphasen-Partialoxidationen bei erhöhter Temperatur (in der Regel einige hundert °C, üblicherweise 100 bis 600°C) durchgeführt. Da die meisten heterogen katalysierten Gasphasen-Partialoxidationen stark exotherm verlaufen, führt man sie häufig zweckmäßigerweise im Wirbelbett oder in Vielkontaktrohr-Festbettreaktoren durch, durch deren die Kontaktrohre umgebenden Raum ein Wärmeaustauschmittel geleitet wird. Der Arbeitsdruck (Absolutdruck) bei heterogen katalysierten Gasphasen- Partialoxidationen kann sowohl unter 1 atm, bei 1 atm oder über 1 atm liegen. In der Regel beträgt er 1 bis 10 atm. Die Zielumsetzung erfolgt während der Verweilzeit des Reaktionsgemisches in der Katalysatorbeschickung, durch die es geleitet wird.

Aufgrund des in der Regel ausgeprägt exothermen Charakters der meisten heterogen katalysierten Gasphasen-Partialoxidationen organischer Verbindungen mit molekularem Sauerstoff werden die Reaktionspartner einerseits üblicherweise mit einem sich unter den Bedingungen der gasphasenkatalytischen Partialoxidationen im wesentlichen inerten Gas verdünnt.

Andererseits besteht im Rahmen einer heterogen katalysierten Gasphasen- Partialoxidation einer organischen Verbindung mit molekularem Sauerstoff insbesondere Interesse an einer hohen Raum-Zeit-Ausbeute der gewünschten Zielverbindung; d.h. es besteht Interesse daran, den Volumenanteil der Reaktionspartner im Ausgangsgemisch, mit dem man den Oxidationsreaktor beschickt, möglichst hoch zu wählen.

Von besonderer Bedeutung ist dabei der Volumenanteil des als Oxidationsmittel zu verwendenden molekularen Sauerstoffs im Beschickungsgas, wie nachfolgend ausgeführt wird.

So ist es einerseits bezüglich der Stöchiometrie der Partialoxidation zur gewünschten Zielverbindung in der Regel erforderlich, den als Oxidationsmittel verwendeten molekularen Sauerstoff in wenigstens stöchiometrischen oder in überstöchiometrischen Mengen einzusetzen (z.B. aus Gründen der Re-Oxidation der als Katalysator eingesetzten oxidischen Masse, sowie zur Minderung von Kohlenstoffabscheidungen).

Andererseits muß der Volumenanteil des als Oxidationsmittel eingesetzten molekularen Sauerstoffs im Beschickungsgasgemisch aus Sicherheitsgründen die sogenannte Sauerstoffgrenzkonzentration unterschreiten.

Unter der Sauerstoffgrenzkonzentration versteht man denjenigen prozentualen Volumenanteil an molekularem Sauerstoff des Beschickungsgasgemisches, bei dessen Unterschreiten unabhängig von der Quantität der Volumenanteile der anderen Bestandteile des Beschickungsgasgemisches, nämlich insbesondere der partiell zu oxidierenden organischen Verbindung sowie dem inerten Verdünnungsgas, eine durch eine örtliche Zündquelle (wie z.B. lokale Überhitzung oder Funkenbildung im Reaktor) eingeleitete Verbrennung z.B. der organischen Verbindung (Explosion) bei gegebenem Druck und Temperatur des Beschickungsgasgemisches sich in selbigem nicht mehr von der Zündquelle her auszubreiten vermag.

Gemäß dem Vorgesagten legt somit die Sauerstoffgrenzkonzentration des Beschickungsgasgemisches den maximalen Volumenanteil des Beschickungsgasgemisches an der partiell zu oxidierenden organischen Verbindung fest und beeinflußt so die erreichbare Raum-Zeit-Ausbeute an Zielprodukt (vgl. EP-A-257 565, S. 5, Zeilen 36/37).

Selbstverständlich wird die Sauerstoffgrenzkonzentration des Beschickungsgasgemisches von der Art der Bestandteile des Beschickungsgasgemisches wesentlich beeinflußt, weshalb der Wahl des inerten Verdünnungsgases (seiner Zusammensetzung) für eine heterogen katalysierte Gasphasen- Partialoxidation einer organischen Verbindung mit molekularem Sauerstoff besondere Bedeutung zukommt.

Die klassischen Verfahren der heterogen katalysierten Gasphasen- Partialoxidation einer organischen Verbindung empfehlen in der Regel die nicht brennbaren Gase Wasserdampf und/oder Stickstoff als inertes Verdünnungsgas zur Ausschaltung des explosiven Bereichs (vgl. z.B. DE-A3 006 894, Seite 6, Zeile 21, DE-A-2 056 614, Seite 2, letzte beiden Zeilen, US-A-4 147 885, Spalte 1, Zeilen 20 bis 35, DE-A-2 547 536, Anspruch 1, DE-A-2 436 818, Seite 2, Absatz 3, DE-A-2 202 734, Seite 4, Zeilen 18 bis 22 und DE-AS-2 009 172, Spalte 4, Zeilen 40 bis 45, wobei die DE-A-2 056 614 die besondere Eignung von Wasserdampf als inertem Verdünnungsgas auf dessen relativ erhöhte molare Wärmekapazität zurückführt (S. 4, Absatz 2, Zeile 4), wohingegen die DE-AS-2 251 364 bezüglich der häufigen Verwendung von Stickstoff als inertem Verdünnungsgas den Kostenaspekt (Luft als Quelle des Oxidationsmittels) anführt).

Die DE-A-1 962 431 betrifft ebenfalls ein Verfahren der heterogen katalysierten Gasphasen-Partialoxidation einer organischen Verbindung mit molekularem Sauerstoff. Als geeignete inerte Verdünnungsgase nennt die DE-A-1 962 431 die nichtbrennbaren Gase Stickstoff, Wasserdampf und Kohlendioxid sowie die brennbaren gesättigten Kohlenwasserstoffe. Ein Ausführungsbeispiel, bei dem ein gesättigter Kohlenwasserstoff als inerter Verdünnungsgasbestandteil verwendet wird, enthält die DE-A-1 962 431 nicht. Die Frage einer Weiterverwertung der im Produktgasgemisch enthaltenen inerten Verdünnungsgasbestandteile wird in der DE-A-1 962 431 nicht angesprochen.

Die DE-A-2 251 364 empfiehlt für ein Verfahren der heterogen katalysierten Gasphasen-Partialoxidation einer organischen Verbindung das nicht brennbare Gas Wasserdampf als inertes Verdünnungsgas, dem das nicht brennbare Gas Stickstoff oder brennbare gesättigte Kohlenwasserstoffe wie Methan, Propan oder Butan zugefügt werden können. Die DE-A-1 468 429 empfiehlt für ein Verfahren der heterogen katalysierten Gasphasen-Partialoxidation einer organischen Verbindung als inerte Verdünnungsgase Kohlendioxid, Stickstoff, gesättigte Kohlenwasserstoffe oder Wasserdampf, wobei Wasserdampf bevorzugt wird.

Weder die DE-A-2 251 364 noch die DE-A-1 468 429 umfassen ein Ausführungsbeispiel, bei dem ein gesättigter Kohlenwasserstoff als brennbarer Verdünnungsgasbestandteil mitverwendet worden wäre. Die Frage einer Weiterverwertung der im Produktgasgemisch enthaltenen inerten Verdünnungsgasbestandteile wird in den vorgenannten beiden Offenlegungsschriften nicht angesprochen.

Die DE-A-3 006 894 betrifft ebenfalls die Problematik, bei einem heterogen katalysierten Gasphasen-Partialoxidationsverfahren einer organischen Verbindung mit molekularem Sauerstoff einerseits das Durchgehen der Reaktion auszuschließen und andererseits eine möglichst hohe Produktivität zu erzielen (S. 2, Zeilen 11 bis 19). Als Lösung empfiehlt sie, das Beschickungsgasgemisch bei geringer Katalysatoraktivität zuzuführen und anschließend längs der Reaktionskoordinate die Katalysatoraktivität sukzessive zu erhöhen. Als inertes Verdünnungsgas benennt die DE-A-3 006 894 die nicht brennbaren Gase Stickstoff, Kohlendioxid und/oder Wasserdampf.

Die DT-AS-1 793 302 betrifft ein Verfahren der heterogen katalysierten Partialoxidation einer organischen Verbindung, bei dem als inertes Verdünnungsgas nach Abtrennung des Zielproduktes das die in der Reaktion erzeugten Kohlenoxide und Wasserdampf enthaltende Reaktionsabgas, d.h. im wesentlichen nicht brennbares Gas, verwendet wird.

Die DE-A-2 056 614 spricht ebenfalls die Problematik des Ausschlusses explosionsartiger Verbrennungsprozesse bei einer heterogen katalysierten Gasphasen-Partialoxidation einer organischen Verbindung an (z.B. S. 3, Absatz 2, letzte zwei Zeilen). Um nachteilige Auswirkungen des bevorzugten Verdünnungsgases Wasserdampf zu vermeiden, empfiehlt die DE-A-2 056 614 die von kondensierbaren Gasen weitgehend befreiten Reaktionsabgase unter teilweisem oder vollständigem Ersatz des Wasserdampfes als inerte Verdünnungsgase in den Oxidationsreaktor zurückzuführen und gleichzeitig das Beschickungsgasgemisch bei niederer Katalysatoraktivität zuzuführen und anschließend die Katalysatoraktivität längs der Reaktionskoordinate sukzessive zu erhöhen. Da das Oxidationsmittel "molekularer Sauerstoff" als Bestandteil von Luft zugeführt wird, sind die effektiven inerten Verdünnungsgase bei der Verfahrensweise der DE-A-2 056 614 im wesentlichen die nicht brennbaren Gase Stickstoff und Kohlendioxid. Die Verfahrensweise der DE-A-2 436 818 entspricht hinsichtlich der verwendeten inerten Verdünnungsgase im wesentlichen jener der DE-A-2 056 614. Dasselbe trifft auf die US-A-4 147 885 zu.

Die DE-A-2 729 841 betrifft ein Verfahren der heterogen katalysierten Gasphasen-Partialoxidation einer organischen Verbindung, das aufgrund der Verwendung eines speziellen Oxidationskatalysators die Möglichkeit eröffnet, anstelle von Wasserdampf als inertes Verdünnungsgas ein im wesentlichen nicht brennbares Gasgemisch aus CO, CO₂, Stickstoff und Argon, das aus dem Produktgasgemisch der heterogen katalysierten Partialoxidation abgetrennt und in das Beschickungsgasgemisch rückgeführt wird, zu verwenden.

Die EP-B-253 409 (vgl. insbesondere S. 5, erste drei Zeilen) und die EP-A-257 565 lehren im Hinblick auf die Vermeidung eines Explosionsrisikos bei einer heterogen katalysierten Gasphasen-Partialoxidation einer organischen Verbindung mit molekularem Sauerstoff die Verwendung von solchen inerten Verdünnungsgasen, die eine erhöhte molare Wärmekapazität Cp aufweisen. Als bevorzugt werden dabei z.B. auf Seite 4, Zeilen 47 ff der EP-B-253 409 sowie auf S.5, Zeilen 26 ff der EP-A-257 565 Gemische aus nicht brennbaren Gasen wie Stickstoff, CO₂, H₂O und brennbaren Gasen wie Methan, Ethan und Propan empfohlen. Neben den genannten Gasen können aber auch noch Helium, Argon, andere gesättigte Kohlenwasserstoffgase, N₂O und Kohlenmonoxid enthalten sein. Als für die Wirkung des inerten Verdünnungsgases wesentlich wird lediglich dessen mittlere molare Wärmekapazität erachtet. So besteht das inerte Verdünnungsgas des Beschickungsgasgemisches in allen Ausführungsbeispielen zu mehr als 55 Vol. -% aus N₂. Ferner empfehlen die EP-B-253 409 und die EP-A-257 565 die im Produktgemisch enthaltenen inerten Verdünnungsgase zumindest teilweise in das Beschickungsgasgemisch zurückzuführen. Die EP-A-361 372 beinhaltet im wesentlichen die gleiche Lehre wie die EP-B-253 409 und die EP-A-257 565.

Das GB-Patent Nr. 1 450 986 empfiehlt, insbesondere wegen seiner erhöhten molaren spezifischen Wärme, die Verwendung von Kohlendioxid als inertes Verdünnungsgas zur Vermeidung der Explosionsgefahr.

Die EP-A-293 224 betrifft ein Verfahren der heterogen katalysierten Gasphasen- Partialoxidation einer organischen Verbindung, bei dem zur Gewährleistung einer sicheren Verfahrensdurchführung die Verwendung eines Kohlendioxid, Wasserdampf und 1 bis 5 C-Atome aufweisende gesättigte Kohlenwasserstoffe enthaltenden Gasgemisches empfohlen wird (S. 3, Zeilen 9 und 20 der EP-A- 293 224). Als wesentlich für die Wirksamkeit des seitens der EP-A-293 224 empfohlenen Inertgasgemisches erachtet die EP-A-293 224 das Beisein von Kohlenoxiden in erhöhten Konzentrationen (S. 3, Zeile 57) sowie eine erhöhte molare Wärmekapazität des Inertgasgemisches (S. 3, Zeile 47). Als weiteren Vorteil der ihrerseits empfohlenen Verfahrensweise erachtet die EP-A-293 224 die Tatsache, daß das zu verwendende Inertgasgemisch zu erheblichen Teilen aus dem Produktgasgemisch der Partialoxidation rekrutiert werden kann. In allen Ausführungsbeispielen umfaßt das im Beschickungsgasgemisch verwendete Inertgasgemisch Wasserdampf und CO₂ in einer Gesamtmenge von wenigstens 15 Vol.-%, bezogen auf das Inertgasgemisch.

Die EP-A-117 146 betrifft ein Verfahren der heterogen katalysierten Gasphasen- Partialoxidation von Propylen zu Acrylsäure, wobei das Propylen durch heterogen katalysierte Dehydrierung von Propan erzeugt wird. Als besonderen Vorteil stellt die EP-A-117 146 heraus, daß man das Produktgasgemisch der Propylendehydrierung ohne Zwischenbehandlung in die Oxidationsstufe überführen und die sich dabei inert verhaltenden Bestandteile anschließend in die Propandehydrierungsstufe rückführen kann. In entsprechender Weise umfaßt das Beschickungsgasgemisch in allen Ausführungsbeispielen ein inertes Verdünnungsgas, das zu mehr als 15 Vol.-% aus Wasserdampf besteht. Die EP-A-372 972 betrifft ein entsprechendes Verfahren zur Herstellung von Acrylnitril, Ethylenoxid und Propylenoxid.

Das US-A-5 312 931 betrifft ein Verfahren der heterogen katalysierten Gasphasen-Partialoxidation von Butadien zu 3,4-Epoxy-1-buten. Als inertes Verdünnungsgas wird Butan eingesetzt. Den Hintergrund dafür bildet die besondere Eignung eines Butan/Butadien flüssig-Gemisches für die absorptive Abtrennung des 3,4-Epoxy-1-butens aus dem Reaktionsgemisch der Gasphasen- Partialoxidation.

Die DE-AS-1 254 137 betrifft ein Verfahren der heterogen katalysierten Gasphasen-Partialoxidation von Ethylen zu Ethylenoxid. Als inertes Verdünnungsgas wird Methan verwendet.

Nachteilig an den Lehren des Standes der Technik ist, daß als inerte Verdünnungsgase u.a. zwar auch solche empfohlen werden, die brennbare Bestandteile enthalten oder ausschließlich aus solchen bestehen, es wurde jedoch nicht erkannt, daß für die Höhe der Sauerstoffgrenzkonzentration eines molekularen Sauerstoff, inertes Verdünnungsgas und partiell zu oxidierende gasförmige organische Substanz enthaltenden Beschickungsgasgemisches weniger die molare Wärmekapazität Cp als vielmehr die Brennbarkeit des inerten Verdünnungsgases von Relevanz ist. Letzterer Sachverhalt ist das Ergebnis intensiver Forschungsarbeit und ohne Anspruch auf Gültigkeit vermutlich darauf zurückzuführen, daß es sich bei Verbrennungsexplosionen in der Regel um radikalische Kettenreaktionen handelt. Offensichtlich ermöglicht nun eine partielle Oxidation einer organischen Verbindung in einer Umgebung aus brennbaren inerten Verdünnungsgasbestandteilen deshalb erhöhte Sauerstoffgrenzkonzentrationen, weil brennbare inerte Verdünnungsgasbestandteile, im Unterschied zu nicht brennbaren inerten Verdünnungsgasbestandteilen, in besonderer Weise zum Abbruch radikalischer Kettenreaktionen befähigt sind (vermutlich infolge Eigenradikalbildung). D.h., je höher der Anteil an brennbaren Bestandteilen im inerten Verdünnungsgas des Beschickungsgasgemisches ist, desto sicherer läßt sich eine heterogen katalysierte Gasphasen-Partialoxidation einer organischen Verbindung mit molekularem Sauerstoff auch bei erhöhten Volumenanteilen der Reaktanten durchführen.

Ein weiterer Nachteil der Verfahren des Standes der Technik ist, daß diejenigen, deren inertes Verdünnungsgas brennbare Bestandteile umfaßt, eine Rückführung der im Produktgasgemisch enthaltenen inerten Verdünnungsgase in die Reaktorbeschickung empfehlen. Dies ist insofern von Nachteil, als während der heterogen katalysierten Gasphasen-Partialoxidation auch nicht brennbare Gase wie H₂O und CO₂ als Nebenprodukte erzeugt oder als Begleiter des Oxidationsmittels molekularer Sauerstoff ins Verfahren eingebracht werden (z.B. N₂ bei Verwendung von Luft als Quelle des Oxidationsmittels). Werden sie mitrückgeführt, geht damit im kontinuierlichen Betrieb eine Minderung der brennbaren Anteile des im Beschickungsgasgemisch enthaltenen inerten Verdünnungsgases einher. Sollen sie nicht mitrückgeführt werden, bedarf es aufwendiger Trennverfahren der im Produktgasgemisch enthaltenen nicht brennbaren inerten Verdünnungsgase von den darin enthaltenen brennbaren inerten Verdünnungsgase oder z.B. der Verwendung einer reinen O₂-Quelle. Darüber hinaus ist eine Kreisführung an inerten Verdünnungsgasen grundsätzlich sowohl auf der investiven Seite als auch auf der Seite der laufenden Betriebskosten nicht unaufwendig. Andererseits bilden brennbare Verdünnungsgasbestandteile im Unterschied zu nicht brennbaren inerten Verdünnungsgasbestandteilen wie CO₂, H₂O oder N₂ Wertstoffe, deren lediglich einmalige Nutzung beim einfachen Durchgang durch den Oxidationsreaktor eine wirtschaftliche Durchführung einer heterogen katalysierten Gasphasen- Partialoxidation einer organischen Verbindung im wesentlichen ausschließt.

Aufgabe der vorliegenden Erfindung war daher ein verbessertes Verfahren der kontinuierlich betriebenen heterogen katalysierten Gasphasen- Partialoxidation einer organischen Verbindung in einem Oxidationsreaktor, dessen Beschickungsgasgemisch neben der partiell zu oxidierenden organischen Verbindung und molekularem Sauerstoff als Oxidationsmittel wenigstens ein sich unter den Bedingungen der heterogen katalysierten Gasphasen-Partialoxidation im wesentlichen inert verhaltendes Verdünnungsgas umfaßt, wobei das sich im wesentlichen inert verhaltende Verdünnungsgas wenigstens zum Teil aus brennbaren Gasen besteht, das die Nachteile der Verfahren des Standes der Technik nicht aufweist.

Demgemäß wurde ein Verfahren der kontinuierlich betriebenen heterogen katalysierten Gasphasen-Partialoxidation einer organischen Verbindung in einem Oxidationsreaktor, dessen Beschickungsgasgemisch neben der partiell zu oxidierenden organischen Verbindung und molekularem Sauerstoff als Oxidationsmittel wenigstens ein sich unter den Bedingungen der heterogen katalysierten Gasphasen-Partialoxidation im wesentlichen inert verhaltendes Verdünnungsgas umfaßt, wobei das sich im wesentlichen inert verhaltende Verdünnungsgas zum Teil aus brennbaren Gasen besteht, gefunden, das dadurch gekennzeichnet ist, daß man nach Durchlaufen des Oxidationsreaktors die im den Oxidationsreaktor verlassenden Produktgasstrom enthaltenen brennbaren Bestandteile des inerten Verdünnungsgases nicht in die heterogen katalysierte Gasphasen-Partialoxidation rückführt, sondern einer Weiterverwendung im Rahmen einer anderen chemischen Umsetzung zuführt.

Als einfaches Beispiel einer Weiterverwendung der brennbaren Bestandteile des inerten Verdünnungsgases kommt deren Weiterleitung in ein Wärme-Kraftwerk in Betracht, wo sie an sich bekannter Weise zur Energiegewinnung verbrannt werden können. Handelt es sich bei den brennenden Bestandteilen des inerten Verdünnungsgases um Kohlenwasserstoffe, so können diese in an sich bekannter Weise auch zur Erzeugung von Synthesegas oder von Acethylen (vgl. z.B. DE-A 4422815) weiterverwendet werden. Eine andere Weiterverwendungskaskade bietet die katalytische Gasphasenoxidation von Propylen zu Acrolein und/oder Acrylsäure im Beisein von Propan und Methan als inertem Verdünnungsgasgemisch. Im Anschluß an die Gasphasenoxidation des Propylens kann das bezüglich dieser Erstumsetzung inerte Propan/Methan-Gemisch einer weiteren katalytischen Gasphasenoxidationsstufe zugeführt werden, in welcher das Propan im Beisein des Methans als inertem Verdünnungsgas zu Acrolein und/oder Acrylsäure partialoxidiert wird. Danach kann das Methan z.B. in einem Wärme-Kraftwerk einer Energie erzeugenden Verbrennung zugeführt werden (die jeweils erforderlichen selektiv wirkenden Katalysatoren sind allgemein bekannt; vgl. z.B. EP-A 608838, EP-A 609112 und EP-A 257565 bzw. EP-B 253409).

Der Vorteil des erfindungsgemäßen Verfahrens liegt u.a. darin begründet, daß der Einsatzstoff "brennbarer Verdünnungsgasbestandteil" bei einfachen Einsatzkosten zweifach genutzt wird:
a) zur Verdünnung des Beschickungsgasgemisches der Gasphasen-Partialoxidation einer organischen Verbindung,
b) z.B. als Brennstoff in einem thermischen Kraftwerk, oder als Ausgangsstoff zur Gewinnung von Synthesegas.

Bezogen auf die konventionellen Einsatzkosten von Brennstoffen in einem thermischen Kraftwerk, oder von Kohlenwasserstoffen als Ausgangsverbindungen für die Synthesegasgewinnung, erfolgt die erfindungsgemäße Verwendung von brennbaren inerten Verdünnungsgasbestandteilen somit kostenneutral. Besonders vorteilhaft ist die erfindungsgemäße Verfahrensweise daher für solche heterogen katalysierten Gasphasen-Partialoxidationen, deren im wesentlichen inertes Verdünnungsgas des Beschickungsgasgemisches im kontinuierlichen Betrieb zu mehr als 85 Vol.-%, vorzugsweise zu wenigstens 90 Vol.-%, besser zu wenigstens 95 Vol.-%, noch besser zu wenigstens 97 Vol.-%, vorteilhaft zu wenigstens 98 Vol.-%, bevorzugt zu wenigstens 99 Vol.-% und am besten zu 100 Vol.-% aus ausschließlich brennbaren inerten Verdünnungsgasbestandteilen besteht. Vorzugsweise umfaßt das inerte Verdünnungsgasgemisch des Beschickungsgasgemisches im Rahmen des erfindungsgemäßen Verfahrens keinen Wasserdampf.

Ferner ist das erfindungsgemäße Verfahren insbesondere für solche heterogen katalysierten Gasphasen-Partialoxidationen einer organischen Verbindung von Vorteil, deren Beschickungsgasgemisch nur aus molekularem Sauerstoff, der partiell zu oxidierenden organischen Verbindung und dem inerten Verdünnungsgas besteht. Im Unterschied dazu umfaßt das Beschickungsgasgemisch bei der Nitrilherstellung in der Regel als weiteren Reaktanten Ammoniak.

Selbstredend bevorzugt das erfindungsgemäße Verfahren innerhalb der brennbaren Verdünnungsgasbestandteile die mehratomigen (>2 Atome), insbesondere aus mehreren C- und H-Atomen aufgebauten Bestandteile, da diese zur Bildung mehrerer rekombinationsfähiger, radikalischer Fragmente befähigt sind. Ferner ist es günstig, wenn der Siedepunkt der inerten Verdünnungsgasbestandteile bei Normaldruck (1 bar) signifikant unterhalb Raumtemperatur (25°C) liegt. D.h., besonders vorteilhafte inerte brennbare Verdünnungsgasbestandteile sind Methan, Ethan, Propan, Butan (n- und/oder iso-) und deren Gemische. Vorzugsweise besteht das inerte Verdünnungsgas des Beschickungsgasgemisches beim erfindungsgemäßen Verfahren zu mehr als 85 Vol.-%, vorzugsweise zu wenigstens 90 Vol.-%, besser zu wenigstens 95 Vol.-%, noch besser zu wenigstens 97 Vol.-%, vorteilhaft zu wenigstens 98 Vol.-%, bevorzugt zu wenigstens 99 Vol.-% und am besten zu 100 Vol.-% aus wenigstens einem der vorgenannten gesättigten Kohlenwasserstoffe. Von besonderem Vorteil ist eine Verwendung von Methan und/oder Propan als inerten Verdünnungsgasbestandteilen. Selbstverständlich kommen aber auch aromatische Kohlenwasserstoffe als brennbare inerte Verdünnungsgase in Betracht.

Für die nachfolgenden heterogen katalysierten Gasphasen-Partialoxidationen eignen sich im Rahmen des erfindungsgemäßen Verfahrens insbesondere die nachfolgenden gesättigten Kohlenwasserstoffe als hauptsächlicher inerter Verdünnungsgasbestandteil:

| Partiell zu oxidierende organische Verbindung | Zielverbindung | bevorzugter Verdünnungsgasbestandteil |
|---|---|---|
| Propylen | Acrolein | Propan |
| Propylen | Acrolein | Methan |
| Propylen | Acrylsäure | Propan |
| Propylen | Acrylsäure | Methan |
| Acrolein | Acrylsäure | Propan |
| Acrolein | Acrylsäure | Methan |
| Ethylen | Ethylenoxid | Ethan oder Methan |
| Butadien | Vinyloxiran | Butan oder Methan |
| iso-Buten | Methacrolein | iso-Butan oder Methan |
| iso-Buten | Methacrylsäure | iso-Butan oder Methan |
| Methacrolein | Methacrylsäure | iso-Butan oder Methan |

Vorzugsweise bilden die angeführten Kohlenwasserstoffe mehr als 85 Vol.-%, vorzugsweise wenigstens 90 Vol.-%, besser wenigstens 95 Vol.-%, noch besser wenigstens 97 Vol.-%, vorteilhaft wenigstens 98 Vol.-%, bevorzugt wenigstens 99 Vol.-% und am besten 100 Vol.-% des inerten Verdünnungsgases des Beschickungsgasgemisches der jeweiligen heterogen katalysierten Gasphasen- Partialoxidation mit molekularem Sauerstoff. Die angeführten Kohlenwasserstoffe sind als inerte Verdünnungsgase für die jeweilige heterogen katalytisierte Gasphasen- Partialoxidation u.a. auch deshalb bevorzugt, weil sie sich entweder besonders inert verhalten oder bei nicht vollständig inertem Verhalten zumindest teilweise zum gewünschten Zeitpunkt umgesetzt werden.

Unabdingbar im Rahmen des erfindungsgemäßen Verfahrens ist die Abtrennung des Zielproduktes aus dem den Oxidationsreaktor verlassenden Produktgasgemisch. In günstigen Fällen kann das dabei verbleibende Restproduktgasgemisch unmittelbar seiner Weiterverwendung (z.B. dem Wärme-Kraftwerk) zugeführt werden. Häufig - insbesondere dann, wenn das erfindungsgemäße Verfahren aus Selektivitätsgründen bei geringen Umsätzen durchgeführt wird - wird man jedoch zuvor nicht umgesetztes Ausgangs- und/oder Zwischenprodukt ebenfalls abtrennen und in der Regel in den Oxidationsreaktor zurückführen. Je nach Bedarf können nicht brennbare inerte Verdünnungsgasbestandteile (auch solche, die im Verlauf der Umsetzung als Nebenprodukte erst gebildet werden) wie N₂, CO₂ oder H₂O vor der Weiterverwendung (z.B. Weiterleitung in das Wärme-Kraftwerk) ebenfalls abgetrennt und z.B. in die Atmosphäre entlassen werden, so daß der Weiterverwendung(z.B. dem Wärme-Kraftwerk) ein im wesentlichen ausschließlich aus den brennbaren Bestandteilen bestehendes Gasgemisch zugeführt wird. Sonstige Nebenprodukte können bei Bedarf vor der Weiterverwendung (z.B. Weiterleitung ins Wärme-Kraftwerk) ebenso abgetrennt werden, wie weniger vorteilhafte brennbare inerte Gase wie CO, die als Reaktionsnebenprodukte ebenfalls im Produktgasgemisch enthalten sein können. Bezüglich der Erzeugung von Synthesegas (Bezeichnung für ein hauptsächlich aus CO und H₂ bestehender Gasgemisch, das als Ausgangsprodukt für synthetische Prozesse dient und im Mengenverhältnis der einzelnen Komponenten den jeweiligen Verwendungszwecken angepaßt werden kann; als Ausgangsprodukte dienen insbesondere Kohlenwasserstoffe, die mit Wasserdampf und Luft oder mit CO₂ bei höheren Temperaturen umgesetzt werden (vgl. z.B. EP-A- 533231)) gilt in entsprechender Weise das gleiche.

Die dabei anzuwendenden Trennverfahren wie fraktionierte Kondensation oder Absorptions-, Extraktionsverfahren sind dem Fachmann bekannt und bedürfen keiner näheren Erläuterung. Die Aufarbeitung und Abtrennung des erwünschten Zielproduktes erfolgt ebenfalls in an sich bekannter Weise.

Als Quelle für den im Rahmen des erfindungsgemäßen Verfahrens als Oxidationsmittel benötigten molekularen Sauerstoff ist Luft lediglich in beschränktem Ausmaß geeignet, da molekularer Sauerstoff in Luft nur mit dem nicht brennbaren inerten Gas N₂ vergesellschaftet vorkommt; d.h. vorzugsweise wird man den für das erfindungsgemäße Verfahren benötigten Sauerstoff einer im wesentlichen reinen Sauerstoffquelle entnehmen.

In ganz besonderer Weise eignet sich das erfindungsgemäße Verfahren zur gasphasenkatalytisch oxidativen Herstellung von Acrolein, Acrylsäure oder deren Gemisch aus Propylen, im mit Multimetalloxid-Katalysatoren beschickten Rohrbündel-Festbettreaktor. Dies um so mehr, als sich Methan (ist bezüglich dieser Umsetzung in besonderem Ausmaß inert) als inertes Verdünnungsgas des Beschickungsgasgemisches für vorgenannte Reaktion gleichzeitig in besonderer Weise eignet, um lokale Überhitzungen ("hot spots") längs der Reaktionsrohre zu vermeiden. Wie dem Fachmann allgemein bekannt ist, läuft die gasphasenkatalytische Oxidation von Propylen zu Acrylsäure mit molekularem Sauerstoff prinzipiell in zwei längs der Reaktionskoordinate aufeinanderfolgenden Schritten ab, von denen der erste zum Acrolein und der zweite von Acrolein zur Acrylsäure führt. Aufgrund dieser Inhärenz schließt eine Eignung des erfindungsgemäßen Verfahrens für die gasphasenkatalytisch oxidative Herstellung von Acrylsäure aus Propylen automatisch eine Eignung für die gasphasenkatalytisch oxidative Herstellung von Acrolein aus Propylen ein, da die Acrylsäureherstellung jederzeit auf der Acroleinstufe unterbrochen werden kann. Ferner eröffnet der Reaktionsablauf in zwei zeitlich aufeinanderfolgenden Schritten die Möglichkeit, die gasphasenkatalytisch oxidative Herstellung von Acrylsäure aus Propylen in zwei hintereinandergeschalteten Oxidationsstufen auszuführen, wobei in jeder der beiden Oxidationsstufen der zu verwendende oxidische Katalysator in optimierender Weise angepaßt werden kann. So wird für die erste Oxidationsstufe (Propylen → Acrolein) in der Regel ein Katalysator auf der Basis von die Elementkombination Mo-Bi-Fe enthaltenden Multimetalloxiden bevorzugt, während für die zweite Oxidationsstufe (Acrolein → Acrylsäure) normalerweise Katalysatoren auf der Basis von die Elementkombination Mo-V enthaltenden Multimetalloxiden bevorzugt werden. Entsprechende Multimetalloxidkatalysatoren für die beiden Oxidationsstufen sind vielfach vorbeschrieben und dem Fachmann wohlbekannt. Beispielsweise verweist die EP-A-253 409 auf Seite 5 auf entsprechende US-Patente. Günstige Katalysatoren offenbaren auch die DE-A-44 31 957 und die DE-A 4431949, insbesondere in Form der Multimetalloxidmassen der allgemeinen Formel I. In der Regel wird das Produktgemisch der ersten Oxidationsstufe ohne Zwischenbehandlung in die zweite Oxidationsstufe überführt. Die einfachste Realisierungsform der beiden Oxidationsstufen bildet daher ein Rohrbündelreaktor, innerhalb dessen sich die Katalysatorbeschickung längs der einzelnen Kontaktrohre mit Beendigung des ersten Reaktionsschrittes entsprechend ändert.

Die beiden Oxidationsstufen können jedoch auch in Form eines aus zwei hintereinandergeschalteten Oxidationsreaktoren bestehenden Oxidationsreaktors realisiert sein. In diesem Fall können auch die übrigen Reaktionsbedingungen, z.B. die Reaktionstemperatur, in der jeweiligen Oxidationsstufe in einfacher Weise optimierend angepaßt werden. Zweckmäßigerweise führt man in diesem Fall den für die zweite Oxidationsstufe benötigten molekularen Sauerstoff erst dem zweiten Oxidationsreaktor zu. Prinzipiell ist die heterogen katalysierte Gasphasen-Partialoxidation von Propylen zu Acrylsäure aber auch einstufig realisierbar. In diesem Fall erfolgen beide Reaktionsschritte in einem Oxidationsreaktor, der mit einem Katalysator bestückt ist und die Umsetzung beider Reaktionsschritte katalysiert. Selbstverständlich kann sich auch die Katalysatorbeschickung innerhalb einer Oxidationsstufe längs der Reaktionskoordinate kontinuierlich oder abrupt ändern. Für die heterogen katalysierte Gasphasenoxidation von Propylen zu Acrolein wird im Beschickungsgasgemisch vorzugsweise eine Propylen/molekularer Sauerstoff Beschickung gewählt, deren Volumenteile sich wie 1(Propylen):1 bis 3 (molekularer Sauerstoff), vorzugsweise wie 1(Propylen):1,5 bis 2(molekularer Sauerstoff), verhalten. Vorgenannte Propylen/molekularer Sauerstoff Beschickung wird mit Vorteil auch für die zweistufige Gasphasenoxidation von Propylen zu Acrylsäure (z.B. in einem aus zwei hintereinandergeschalteten Oxidationsreaktoren bestehenden Oxidationsreaktor) gewählt. In der Regel wird dabei das die erste Stufe (den ersten Oxidationsreaktor) verlassende Produktgasgemisch ohne Zwischenbehandlung in den zweiten Oxidationsreaktor überführt. Zweckmäßigerweise führt man der zweiten Oxidationsstufe (dem zweiten Oxidationsreaktor) zusätzlich molekularen Sauerstoff als Oxidationsmittel zu. Die Menge an zusätzlich zugeführtem molekularem Sauerstoff wird vorzugsweise so gewählt, daß auch das Beschickungsgasgemisch der zweiten Oxidationsstufe (des zweiten Oxidationsreaktors) eine wenigstens stöchiometrische bis etwa zweifach stöchiometrische Menge an O₂ umfaßt. Dabei wird man wie für die erste Oxidationsstufe den Sauerstoff vorzugsweise ebenfalls einer im wesentlichen reinen Sauerstoffquelle entnehmen. Bei Bedarf kann man aus dem die erste Oxidationsstufe (den ersten Oxidationsreaktor) verlassenden Produktgasgemisch in der ersten Oxidationsstufe als Nebenprodukte gebildeten Wasserdampf und CO₂ abtrennen bevor man es in die zweite Oxidationsstufe (in den zweiten Oxidationsreaktor) überführt.

Der Unterschied des erfindungsgemäßen Verfahrens zu den Verfahren des Standes der Technik besteht insbesondere darin, daß das erfindungsgemäße Verfahren auf wirtschaftliche Art und Weise einen erhöhten Anteil an brennbaren Bestandteilen im inerten Verdünnungsgas des Beschickungsgasgemisches kontinuierlich betriebener heterogen katalysierter Gasphasen-Partialoxidationen organischer Verbindungen mit molekularem Sauerstoff ermöglicht. Dieser erhöhte Anteil an brennbaren Bestandteilen im inerten Verdünnungsgas des Beschickungsgasgemisches ist jedoch die Voraussetzung für die sichere Durchführung heterogen katalysierter Gasphasen-Partialoxidationen organischer Verbindungen, insbesondere mit erhöhten Reaktantenvolumenanteilen des Beschickungsgasgemisches. Letzteres bildet die Grundlage für erhöhte Raum-Zeit-Ausbeuten. So sind bei Verwendung von Methan und/oder Propan als inertes Verdünnungsgas des Beschickungsgasgemisches für die Herstellung von Acrolein und/oder Acrylsäure Beschickungsgasgemische mit erhöhter Sicherheit handhabbar, deren Propylenbeschickung >30 Vol.-% bis zu 40 oder 45 Vol.-%, bezogen auf das Beschickungsgasgemisch, beträgt. Günstige Beschickungsgemische sind auch solche, die umfassen:
15 bis 30 Vol.-% Propylen,
20 bis 40 Vol.-% Sauerstoff und
30 bis 65 Vol.-% Methan und/oder Propan.

Für die Methacrolein- und/oder Methacrylsäureherstellung gilt entsprechendes. Insbesondere eignet sich die erfindungsgemäße Verfahrensweise auch zur gasphasenkatalytisch oxidativen Herstellung von Phthalsäureanhydrid, insbesondere aus o-Xylol.

### Beispiele (Einfluß der Brennbarkeit der inerten Verdünnungsgasbestandteile auf die Sauerstoffgrenzkonzentration)

Ermittlung der Sauerstoffgrenzkonzentration von bei einer Ausgangstemperatur von 250°C und einem Ausgangsdruck von 1 bar befindlichen Beschickungsgasgemischen, bestehend aus Propylen (partiell zu oxidierende organische Verbindung), molekularem Sauerstoff (Oxidationsmittel) und einem bezüglich einer heterogen katalysierten Gasphasen-Partialoxidation des Propylens zu Acrylsäure inerten Verdünnungsgas.

### Allgemeine Versuchsdurchführung:

Die Versuche wurden in einem geschlossenen kugelförmigen 5l-Hochdruckbehälter aus Edelstahl durchgeführt. Die Erzeugung des Gasgemisches in dem anfangs evakuierten Hochdruckbehälter erfolgte nach der Partialdruckmethode. Nach einer Mischzeit von 10 min mittels eines Magnetrührwerkes wurde versucht, das Gasgemisch mittels eines durchschmelzenden Platindrahtes zu zünden. Eine dadurch eventuell ausgelöste selbständige Ausbreitung einer Reaktionsfront (Explosion) wurde über den zeitlichen Anstieg des Behälterinnendruckes (Messung mit piezoelektrischem Druckaufnehmer) und über die Erhöhung der Temperatur im Behälter detektiert.

Ergebnisse (die zugrunde gelegten spezifischen molaren Wärmen Cp fußen auf den Daten aus "Ihsan Barin, Thermochemical Data of Pure Substances, Part I u. Part II, VCH Verlagsgesellschaft, Weinheim, Zweite Auflage, 1993", wobei bei Gasgemischen ideales Gasverhalten angenommen wurde):
a) Ausschließliche Verwendung von Methan als inertes brennbares Verdünnungsgas. D.h. das inerte Verdünnungsgas bestand ausschließlich aus brennbaren Bestandteilen. Die spezifische molare Wärme Cp von Methan beträgt unter den vorgegebenen Bedingungen 47,5 J/mol·K. Die ermittelte Sauerstoffgrenzkonzentration beträgt 32 Vol.-%.
   D.h. in einem Gemisch aus Propylen, molekularem O₂ und Methan als Inertgas, das sich bei 250°C und 1 bar befindet, vermag sich eine lokale Entzündung (Explosion) unabhängig von der speziellen Gemischzusammensetzung dann nicht mehr selbständig auszubreiten, wenn der Volumenanteil an O₂ im Gesamtgemisch < 32 Vol.-% beträgt. D.h. in einem bei 1 bar und 250°C befindlichen Gemisch aus 31 Vol.-% O₂, 20 Vol.-% Propylen und 49 Vol.-% Methan vermag sich eine lokale Entzündung nicht mehr selbständig auszubreiten.
b) Verwendung eines 3,2 (Propan) : 96,8 (CO₂)-Gemisches (Verhältnis der Volumenanteile) aus Propan und Kohlendioxid als inertes Verdünnungsgas. D.h. das inerte Verdünnungsgas bestand fast ausschließlich aus nicht brennbarem Verdünnungsgas. Die Zusammensetzung des Inertgasgemisches wurde so gewählt, damit es unter den vorgegebenen Bedingungen ebenfalls ein Cp von 47,5 J/mol·K aufweist. Die ermittelte Sauerstoffgrenzkonzentration beträgt lediglich 15 Vol.-%.
   D.h. in einem unter entsprechenden Bedingungen wie in a) befindlichen Gemisch aus 31 Vol.-% O₂, 20 Vol.-% Propylen und 49 Vol.-% des inerten Verdünnungsgases breitet sich eine lokale Entzündung selbständig aus.
c) Verwendung eines 48,3 (Propan) : 51,7 (Methan)-Gemisches (Verhältnis der Volumenanteile) aus Propan und Methan als inertes Verdünnungsgas. D.h. das inerte Verdünnungsgas bestand ausschließlich aus brennbaren Bestandteilen. Die spezifische molare Wärme Cp dieses Gemisches beträgt unter den vorgegebenen Bedingungen = 80,8 J/mol·K. Die ermittelte Sauerstoffgrenzkonzentration beträgt 37 Vol.-%.
d) Verwendung eines 50 (Propan) : 50 (CO₂)-Gemisches (Verhältnis der Volumenanteile) aus Propan und Kohlendioxid als inertes Verdünnungsgas. D.h. das inerte Verdünnungsgas enthielt auch nicht brennbare Bestandteile. Die Zusammensetzung des Inertgasgemisches wurde so gewählt, damit es unter den vorgegebenen Bedingungen ebenfalls ein Cp von 80,8 J/mol·K aufweist.
   Die ermittelte Sauerstoffgrenzkonzentration beträgt lediglich 34 Vol.-%. D.h. trotz signifikant höherem Cp-Wert des inerten Verdünnungsgases im Vergleich zu a), liegt die Sauerstoffgrenzkonzentration nur bei einem vergleichbaren Volumenanteil wie bei a).

## Patentansprüche

1. Verfahren der kontinuierlich betriebenen heterogen katalysierten Gasphasen-Partialoxidation einer organischen Verbindung in einem Oxidationsreaktor, dessen Beschickungsgasgemisch neben der partiell zu oxidierenden organischen Verbindung und molekularem Sauerstoff als Oxidationsmittel wenigstens ein sich unter den Bedingungen der heterogen katalysierten Gasphasen-Partialoxidation im wesentlichen inert verhaltendes Verdünnungsgas umfaßt, wobei das sich im wesentlichen inert verhaltende Verdünnungsgas zum Teil aus brennbaren Gasen besteht, dadurch gekennzeichnet, daß man nach Durchlaufen des Oxidationsreaktors die im den Oxidationsreaktor verlassenden Produktgasstrom enthaltenen brennbaren Bestandteile des inerten Verdünnungsgases nicht in die heterogen katalysierte Gasphasen-Partialoxidation rückführt, sondern einer Weiterverwendung im Rahmen einer anderen chemischen Umsetzung zuführt.

2. Verfahren nach Anspruch 1, dadurch gekennzeichnet, daß man die im den Oxidationsreaktor verlassenden Produktgasstrom enthaltenen brennbaren Bestandteile des inerten Verdünnungsgases in ein Wärme-Kraftwerk weiterleitet und dort zur Energiegewinnung verbrennt.

3. Verfahren nach Anspruch 1, dadurch gekennzeichnet, daß es sich bei den im den Oxidationsreaktor verlassenden Produktgasstrom enthaltenen brennbaren Bestandteilen des inerten Verdünnungsgases um Kohlenwasserstoffe handelt, die zur Erzeugung von Synthesegas weiterverwendet werden.

4. Verfahren nach Anspruch 1, dadurch gekennzeichnet, daß es sich bei den im den Oxidationsreaktor verlassenden Produktgasstrom enthaltenen brennbaren Bestandteilen des inerten Verdünnungsgases um Kohlenwasserstoffe handelt, die zur Erzeugung von Acethylen weiterverwendet werden.

5. Verfahren nach Anspruch 1 bis 4, dadurch gekennzeichnet, daß das Beschickungsgasgemisch des Oxidationsreaktors aus Propylen, molekularem Sauerstoff und einem zu mehr als 85 Vol.-% aus wenigstens einem der gesättigten Kohlenwasserstoffe Methan, Ethan, Propan und Butan bestehenden Verdünnungsgas besteht.

## Claims

1. A process for the continuous heterogeneously catalyzed gas-phase partial oxidation of an organic compound in an oxidation reactor, whose feed gas mixture comprises, apart from the organic compound to be partially oxidized and molecular oxygen as oxidant, at least one diluent gas which is essentially inert under the conditions of the heterogeneously catalyzed gas-phase partial oxidation, where the essentially inert diluent gas consists partly of combustible gases, wherein, after passage through the oxidation reactor, the combustible constituents of the inert diluent gas present in the product gas stream leaving the oxidation reactor are not recirculated to the heterogeneously catalyzed gas-phase partial oxidation, but are put to further use for the purposes of another chemical reaction.

2. A process as claimed in claim 1, wherein the combustible constituents of the inert diluent gas present in the product gas stream leaving the oxidation reactor are passed on to a thermal power station and are there burned for energy recovery.

3. A process as claimed in claim 1, wherein the combustible constituents of the inert diluent gas present in the product gas stream leaving the oxidation reactor are hydrocarbons which are further used for the production of synthesis gas.

4. A process as claimed in claim 1, wherein the combustible constituents of the inert diluent gas present in the product gas stream leaving the oxidation reactor are hydrocarbons which are further used for the production of acetylene.

5. A process as claimed in any of claims 1 to 4, wherein the feed gas mixture for the oxidation reactor comprises propylene, molecular oxygen and a diluent gas comprising more than 85% by volume of at least one of the saturated hydrocarbons methane, ethane, propane and butane.

## Revendications

1. Procédé d'oxydation partielle en phase gazeuse, à catalyse hétérogène, en continue, d'un composé organique dans un réacteur d'oxydation, dont le mélange de gaz de charge comporte au moins un gaz de dilution essentiellement inerte dans les conditions de l'oxydation partielle en phase gazeuse à catalyse hétérogène, en plus du composé organique à oxyder de façon partielle et de l'oxygène moléculaire en tant qu'agent oxydant, le gaz de dilution essentiellement inerte étant constitué pour partie de gaz combustibles, caractérisé en ce que l'on ne recycle pas, après traversée du réacteur d'oxydation, la partie combustible du gaz de dilution inerte contenue dans le courant gazeux de produits sortant du réacteur d'oxydation dans l'oxydation partielle en phase gazeuse à catalyse hétérogène, mais en ce qu'on l'utilise ensuite dans le cadre d'une autre réaction chimique.

2. Procédé selon la revendication 1, caractérisé en ce que l'on transfère la partie combustible du gaz de dilution inerte contenue dans le courant gazeux de produits sortant du réacteur d'oxydation vers une centrale thermique et en ce qu'on l'y brûle pour produire de l'énergie.

3. Procédé selon la revendication 1, caractérisé en ce que les parties combustibles du gaz de dilution inerte contenues dans le courant gazeux de produits sortant du réacteur d'oxydation sont des hydrocarbures qu'on utilise ensuite pour la production de gaz de synthèse.

4. Procédé selon la revendication 1, caractérisé en ce que les parties combustibles du gaz de dilution inerte contenues dans le courant gazeux de produits sortant du réacteur d'oxydation sont des hydrocarbures qu'on utilise ensuite pour la production d'acétylène.

5. Procédé selon l'une quelconque des revendications 1 à 4, caractérisé en ce que le mélange de gaz de charge du réacteur d'oxydation est constitué de propylène, d'oxygène moléculaire et d'un gaz de dilution constitué de plus de 85% en volume d'au moins un des hydrocarbures saturés du groupe du méthane, de l'éthane, du propane, et du butane.
